# EUROPÄISCHE PATENTSCHRIFT

(11) **EP 2 834 644 B1**
(45) Veröffentlichungstag und Bekanntmachung des Hinweises auf die Patenterteilung: **21.08.2019**
(21) Anmeldenummer: 13719436.1
(22) Anmeldetag: 05.04.2013
(51) Int. Cl.: G01N 33/68, C07K 7/04

(54) **POLYMERE, ENTHALTEND MULTIVALENTE AMYLOID-BETA-BINDENDE D-PEPTIDE UND DEREN VERWENDUNG**
POLYMERS CONTAINING MULTIVALENT AMYLOID-BETA-BINDING D-PEPTIDES AND THEIR USE
POLYMÈRES CONTENANT DES PEPTIDES D MULTIVALENTS LIANT DES BÊTA-AMYLOÏDES ET LEUR UTILISATION

(30) Priorität: 05.04.2012 DE 102012102998; 14.09.2012 DE 102012108598; 14.09.2012 DE 102012108599
(43) Veröffentlichungstag der Anmeldung: 11.02.2015
(60) Teilanmeldung: 19170316.4
(73) Patentinhaber: Forschungszentrum Jülich GmbH, 52425 Jülich (DE)
(72) Erfinder: WILLBOLD, Dieter, 52428 Jülich (DE); FUNKE, Susanne Aileen, 96242 Sonnefeld (DE); BRENER, Oleksander, 40591 Düsseldorf (DE); NAGEL-STEGER, Luitgard, 40764 Langenfeld (DE); BARTNIK, Dirk, 51105 Köln (DE)
(74) Vertreter: Fitzner, Uwe
(86) Internationale Anmeldenummer: PCT/EP2013/057161
(87) Internationale Veröffentlichungsnummer: WO 2013/150127

(56) Entgegenhaltungen:
- WO-A2-02/42462
- WO-A2-2007/047967
- WO-A2-2011/147797
- ZHANG GUOBAO ET AL: "Multiple-peptide conjugates for binding beta-amyloid plaques of Alzheimer's disease", BIOCONJUGATE CHEMISTRY, ACS, WASHINGTON, DC, US, Bd. 14, Nr. 1, 1. Januar 2003 (2003-01-01) , Seiten 86-92, XP002467772, ISSN: 1043-1802, DOI: 10.1021/BC025526I
- KATJA WIESEHAN ET AL: "Selection of D-Amino-Acid Peptides That Bind to Alzheimer's Disease Amyloid Peptide A[beta]142 by Mirror Image Phage Display", CHEMBIOCHEM, Bd. 4, Nr. 8, 4. August 2003 (2003-08-04), Seiten 748-753, XP55069247, ISSN: 1439-4227, DOI: 10.1002/cbic.200300631
- SIDHARTHA M. CHAFEKAR ET AL: "Branched KLVFF Tetramers Strongly Potentiate Inhibition of [beta]-Amyloid Aggregation", CHEMBIOCHEM, Bd. 8, Nr. 15, 15. Oktober 2007 (2007-10-15), Seiten 1857-1864, XP55052606, ISSN: 1439-4227, DOI: 10.1002/cbic.200700338
- STAINS CLIFF I ET AL: "Molecules that target beta-amyloid", CHEMMEDCHEM, WILEY - VCH VERLAG., WEINHEIM, DE, Bd. 2, Nr. 12, 1. Dezember 2007 (2007-12-01), Seiten 1675-1692, XP009107501, ISSN: 1860-7179, DOI: 10.1002/CMDC.200700140
- FUNKE SUSANNE AILEEN ET AL: "Peptides for Therapy and Diagnosis of Alzheimer's Disease", CURRENT PHARMACEUTICAL DESIGN, BENTHAM SCIENCE PUBLISHERS, NL, Bd. 18, Nr. 6, 1. Februar 2012 (2012-02-01), Seiten 755-767, XP009170739, ISSN: 1381-6128
- SUSANNE AILEEN FUNKE ET AL: "Mirror image phage display-a method to generate d-peptide ligands for use in diagnostic or therapeutical applications", MOLECULAR BIOSYSTEMS, Bd. 5, Nr. 8, 1. Januar 2009 (2009-01-01), Seite 783, XP55069250, ISSN: 1742-206X, DOI: 10.1039/b904138a

## Beschreibung

Die vorliegende Erfindung betrifft neue multivalente Amyloid-Beta-bindende polymere Substanzen, bestehend aus mehreren miteinander verbundenen Substanzen, die für sich bereits Amyloid-Beta-bindende Eigenschaften haben, sowie die Verwendung dieser im Folgenden "Polymere" genannten Substanzen insbesondere in der Medizin.

Aufgrund der demographischen Entwicklung in den nächsten Jahrzehnten wird sich die Zahl der Personen, die an altersbedingten Erkrankungen leiden, erhöhen. Hier ist insbesondere die sog. Alzheimer-Krankheit (AD, Alzheimersche Demenz, lateinisch = Morbus Alzheimer) zu nennen.

Bisher existiert kein Wirkstoff oder Medikament, das gegen die Ursachen von AD wirkt. Die bisher eingesetzten und zugelassenen Medikamente mildern einige der bei der Alzheimerschen Demenz auftretenden Symptome. Sie sind aber nicht in der Lage, den Krankheitsfortschritt zu verlangsamen oder eine Heilung herbeizuführen. Es existieren einige Substanzen, die im Tierversuch Erfolge bei der Prävention, aber nicht (unbedingt) bei der Behandlung von AD gezeigt haben. Wirkstoffe gegen neurodegenerative Erkrankungen sind aus der DE 10 2006 015 140 A1 bekannt.

Ein Merkmal der Alzheimer-Erkrankung sind extrazelluläre Ablagerungen des Amyloid-Beta-Peptids (A-Beta-Peptid, Aß oder Aß-Peptid). Diese Ablagerung des A-Beta-Peptids in Plaques ist typischerweise in den Gehirnen von AD-Patienten *post mortem* festzustellen. Deshalb werden verschiedene Formen des A-Beta-Peptids - wie z.B. Fibrillen - für die Entstehung und das Fortschreiten der Krankheiten verantwortlich gemacht. Zusätzlich werden seit einigen Jahren die kleinen, frei diffundierbaren A-Beta-Oligomere als hauptsächliche Verursacher der Entstehung und des Fortschritts der AD gesehen.

A-Beta-Monomere, als Bausteine der A-Beta-Oligomere, entstehen im menschlichen Körper ständig und sind vermutlich *per se* nicht toxisch. Es besteht sogar die Möglichkeit, dass Monomere eine positive Funktion besitzen. A-Beta-Monomere können sich in Abhängigkeit von ihrer Konzentration zufällig zusammen lagern. Die Konzentration ist abhängig von ihrer Bildungs- und Abbaurate im Körper. Findet mit zunehmendem Alter eine Erhöhung der Konzentration an A-Beta-Monomeren im Körper statt, ist eine spontane Zusammenlagerung der Monomere zu A-Beta-Oligomeren immer wahrscheinlicher. Die so entstandenen A-Beta-Oligomere könnten sich analog zu den Prionen vermehren und letztendlich zur Morbus Alzheimer-Krankheit führen.

Ein wichtiger Unterschied zwischen Prävention und Behandlung oder gar Heilung der AD liegt in der Tatsache, dass eine Prävention möglicherweise schon durch die Verhinderung der Bildung der ersten A-Beta-Oligomere erreicht werden kann. Hierzu sind einige, wenige A-Beta-Liganden ausreichend, die wenig affin und selektiv bezüglich der A-Beta-Oligomere sind.
Die Bildung der A-Beta-Oligomere aus vielen Monomeren ist eine Reaktion hoher Ordnung und damit in hoher Potenz von der A-Beta-Monomer-Konzentration abhängig. Somit führt schon eine kleine Verringerung der aktiven A-Beta-Monomer-Konzentration zu einer Verhinderung der Bildung der ersten A-Beta-Oligomere. Auf diesem Mechanismus basieren vermutlich die bisher sich in der Entwicklung befindlichen eher präventiven Therapie-Konzepte und Substanzen.
Bei der Behandlung von AD ist jedoch von einer völlig veränderten Situation auszugehen. Hier liegen A-Beta-Oligomere oder evtl. auch schon größere Polymere oder Fibrillen vor, die sich durch Prionen-ähnliche Mechanismen vermehren. Diese Vermehrung ist jedoch eine Reaktion niederer Ordnung und ist kaum noch von der A-Beta-Monomer-Konzentration abhängig.

Die aus dem Stand der Technik bekannten Substanzen verringern die Konzentration von A-Beta-Monomeren und/oder Oligomeren auf verschiedenste Art und Weise. So sind z.B. Gamma-Sekretase-Modulatoren bekannt, die im Tierversuch zur Prävention eingesetzt wurden.
Aus der WO 02/081505 A2 sind verschiedene Sequenzen von D-Aminosäuren bekannt, die an A-Beta-Peptide binden. Diese Sequenzen aus D-Aminosäuren binden mit einer Dissoziationskonstante (K_{D}-Wert) von 4 µM an Amyloid-Beta-Peptide.
Aus der WO 2011/147797 A2 sind Hybridverbindungen bekannt, bestehend aus Aminopyrazolen und Peptiden, die A-Beta-Oligomerisation verhindern.

Verbindungen die mit A-Beta-Peptiden interagieren sind aus der DE 10 2008 037 564 A1, DE 696 21 607 T2 oder DE 10 2010 019 336 A1 bekannt. Die Bindung eines multivalenten Polymers an zwei Bindungspartner wird in der WO 2008/116293 A1 beschrieben.

Zhang G. et al. Bioconjugate Chemistry, 14(1), 2003 offenbart, dass die Bildung von β-Amyloid-Plaques bei Alzheimer durch den intermolekularen Kontakt der 5-Aminosäuresequenz KLVFF in β-Amyloid-Peptiden mit einer Größe von 40 bis 43 Resten ausgelöst wird.

Wiesehahn et al, CHEMBIOCHEM, 4(8), 2003 offenbart einen Phagen-Display-Assay, um neue und hochspezifische Liganden für das Alzheimer-Peptid Aβ(1-42) zu identifizieren.

Sidhartha et al. ChemBioChem, 8(15), 2007 offenbart ein Dendrimer aus vier Peptiden mit dem Motiv KLVFF.

Stains et al, CHEMMEDCHEM, 2(12) 2007 betrifft das aktuelle Wissen in Bezug auf Moleküle, die nachweislich mit oligomeren oder fibrillären Formen des beta-Amyloid-Peptids interagieren.

Die WO 2007/047967 A2 betrifft eine Vorrichtung, die innerhalb eines Patienten mit Alzheimer-Krankheit (AD) platziert wird, um neurotoxische beta-Amyloidpeptide (nt-bAP) aus Körperflüssigkeiten zu extrahieren.

Die WO 02/42462 A2 betrifft therapeutische Mittel, die zur Behandlung einer amyloidogenen Störung geeignet sind.

Die WO 2011/147797 A2 betrifft eine Hybridverbindung auf der Basis von Aminopyrazolderivaten und Peptiden zur Verwendung als Therapeutikum bei der Behandlung von Krankheiten.

Bei vielen Substanzen, die im Tierversuch positive Ergebnisse gezeigt haben, konnte diese Wirkung in klinischen Studien am Menschen nicht bestätigt werden. In klinischen Studien der Phase II und III dürfen nur Menschen behandelt werden, die eindeutig mit AD diagnostiziert wurden. Hier reicht eine geringe Verringerung der A-Beta-Monomerkonzentration nicht mehr aus, um zu verhindern, dass sich aus den bereits vorhandenen A-Beta-Oligomeren noch mehr bilden, z.B. durch einen Prion-ähnlichen Mechanismus. Die Vermehrung der A-Beta-Oligomere oder noch besser ihre Zerstörung oder Unschädlichmachung ist aber unbedingt erforderlich, um Einfluss auf den Krankheitsverlauf zu nehmen.

Bisher wird die Alzheimersche Demenz hauptsächlich durch neuro-psychologische Tests diagnostiziert, durch Experimente an Personen, bei denen Demenzsymptome erkannt wurden. Es ist jedoch bekannt, dass A-Beta-Oligomere und die zeitlich darauf folgenden Fibrillen und Plaques bis zu 20 Jahre vor dem Auftreten der Symptome im Gehirn der Patienten entstehen, und schon irreversible Schäden verursacht haben können. Allerdings gibt es bisher noch keine Möglichkeit, die AD vor dem Ausbruch der Symptome zu diagnostizieren.

Es besteht somit weiterhin ein Bedarf an neuen Verbindungen (Wirkstoffen), die sehr spezifisch und mit hoher Affinität an A-Beta-Oligomere binden, und so deren Vermehrung verhindern. Diese Verbindungen sollten keine Effekte mit unerwünschten Nebenwirkungen zeigen, insbesondere keine Immunreaktion hervorrufen. Die Verbindungen sollen außerdem toxische A-Beta-Oligomere und damit auch die kleinen frei diffundierbaren Oligomere auch in kleinen Konzentrationen erkennen, vollständig vernichten und/oder deren (Prionen-ähnliche) Vermehrung verhindern.

Des Weiteren besteht auch ein Bedarf an neuen Verbindungen, die als Sonden für die Erkennung und Markierung von A-Beta-Oligomeren einsetzbar sein, insbesondere, wenn diese Oligomere erst in kleinen Konzentrationen auftreten.

Diese Aufgabe wird durch die erfindungsgemäßen Polymere, enthaltend mindestens zwei Substanzen (Monomere), die an Amyloid- Beta-Oligomere binden, wobei eine Substanz (Monomer) der mindestens zwei Substanzen SEQ ID NO: 1 (RD2) ist, gelöst..

Im Sinne der vorliegenden Erfindung bezeichnet der Begriff A-Beta-Oligomere sowohl A-Beta-Aggregate als auch A-Beta-Oligomere und auch kleine frei diffundierende A-Beta-Oligomere. Polymere im Sinne der Erfindung ist eine Substanz gebildet aus 2, 3, 4, 5, 6, 7, 8, 9, 10, 11, 12, 13, 14, 15, 16, 17, 18, 19 oder 20 für sich bereits Amyloid-Beta-bindenden Substanzen (Monomeren) oder Vielfachen davon.

In einer Ausführung handelt es sich bei den Polymeren um Peptide. Diese bestehen bevorzugt im Wesentlichen aus D-Aminosäuren.

Im Sinne der vorliegenden Erfindung bedeutet der Begriff "im Wesentlichen aus D-Aminosäuren", dass die einzusetzenden Monomere mindestens 60%, bevorzugt 75%, 80%, besonders bevorzugt 85%, 90%, 95%, insbesondere 96%, 97%, 98%, 99%, 100% aus D-Aminosäuren aufgebaut sind.

In einer Variante der Erfindung werden Monomere eingesetzt, die an ein A-Beta-Monomer und/oder A-Beta-Oligomere und/oder Fibrillen des A-Beta-Peptids mit einer Dissoziationskonstante (K_{D}-Wert) von höchstens 500 µM, bevorzugt 250, 100, 50 µM, besonders bevorzugt 25, 10, 6 µM, insbesondere 4, 2, 1 µM bindet.

Das erfindungsgemäße Polymer enthält 2, 3, 4, 5, 6, 7, 8, 9, 10 oder mehr der oben beschriebenen Monomere.

In einer weiteren Ausführung werden die Monomere ausgewählt aus der Gruppe bestehend aus:
SEQ ID NO:1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12, SEQ ID NO:13, SEQ ID NO:14, SEQ ID NO:15, SEQ ID NO:16, SEQ ID NO:17, SEQ ID NO:18, SEQ ID NO:19, SEQ ID NO:20, SEQ ID NO:21, SEQ ID NO:22, SEQ ID NO:23, SEQ ID NO:24, SEQ ID NO:25, SEQ ID NO:26, SEQ ID NO:27, SEQ ID NO:28, SEQ ID NO:29, SEQ ID NO:30, SEQ ID NO:31, SEQ ID NO:32, SEQ ID NO:33, SEQ ID NO:34, SEQ ID NO:35, SEQ ID NO:36, SEQ ID NO:37, SEQ ID NO:38, SEQ ID NO:39, SEQ ID NO:40, SEQ ID NO:41, SEQ ID NO:42, SEQ ID NO:43, SEQ ID NO:44, SEQ ID NO:45, SEQ ID NO:46, SEQ ID NO:47, SEQ ID NO:48, SEQ ID NO:49, SEQ ID NO:50, SEQ ID NO:51, SEQ ID NO:52, SEQ ID NO:53, SEQ ID NO:54, SEQ ID NO:55, SEQ ID NO:56, SEQ ID NO:57, SEQ ID NO:58, SEQ ID NO:59, SEQ ID NO:60, SEQ ID NO:61, SEQ ID NO:62, SEQ ID NO:63, SEQ ID NO:64. SEQ ID NO:65, SEQ ID NO:66, SEQ ID NO:67, SEQ ID NO:68, SEQ ID NO:69, SEQ ID NO:70, SEQ ID NO:71, SEQ ID NO:72, SEQ ID NO:73, SEQ ID NO:74, SEQ ID NO:75, SEQ ID NO:76, SEQ ID NO:77, SEQ ID NO:78 und SEQ ID NO:79..

In einer weiteren Variante binden die erfindungsgemäßen Polymere an Teile des Amyloid beta-Peptids.

In einer weiteren nicht erfindungsgemäßen Variante weisen die Monomere Sequenzen auf, die sich von den angegebenen Sequenzen um bis zu drei Aminosäuren unterscheiden.
Ferner werden als Monomere auch Sequenzen eingesetzt, die die oben genannten Sequenzen enthalten.

In einer weiteren Variante weisen die Monomere Fragmente der oben genannten Sequenzen auf oder weisen homologe Sequenzen zu den oben genannten Sequenzen auf.

"Homologe Sequenzen" oder "Homologe" bedeutet im Sinne der Erfindung, dass eine Aminosäuresequenz eine Identität mit einer der oben genannten Aminosäuresequenz der Monomere von mindestens 50, 55, 60, 65, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 ,100 % aufweist. Anstelle des Begriffs "Identität" werden in der vorliegenden Beschreibung die Begriffe "homolog" oder "Homologie" gleichbedeutend verwendet. Die Identität zwischen zwei Nukleinsäuresequenzen oder Polypeptidsequenzen wird durch Vergleich mit Hilfe des Programms BESTFIT basierend auf dem Algorithmus von Smith, T.F. und Waterman, M.S (Adv. Appl. Math. 2: 482-489 (1981)) berechnet unter Einstellung folgender Parameter für Aminosäuren: Gap creation penalty: 8 und Gap extension penalty: 2; und folgender Parameter für Nukleinsäuren: Gap creation penalty: 50 und Gap extension penalty: 3. Bevorzugt wird die Identität zwischen zwei Nukleinsäuresequenzen oder Polypeptidsequenzen durch die Identität der Nukleinsäuresequenz / Polypeptidequenz über die jeweils gesamte Sequenzlänge definiert, wie sie durch Vergleich mit Hilfe des Programms GAP basierend auf dem Algorithmus von Needleman, S.B. und Wunsch, C.D. (J. Mol. Biol. 48: 443-453) unter Einstellung folgender Parameter für Aminosäuren berechnet wird: Gap creation penalty: 8 und Gap extension penalty: 2; und die folgenden Parameter für Nukleinsäuren Gap creation penalty: 50 und Gap extension penalty: 3. Zwei Aminosäuresequenzen sind im Sinne der vorliegenden Erfindung identisch wenn sie die selbe Aminosäuresequenz besitzen.

Unter Homologe sind in einer Variante die entsprechenden retro-inversen Sequenzen der oben genannten Monomere zu verstehen. Mit dem Begriff "retro-inverse Sequenz" wird erfindungsgemäß eine Aminosäuresequenz bezeichnet, die sich aus Aminosäuren in der enantiomeren Form zusammensetzt (invers: Chiralität des alpha-C-Atoms invertiert) und bei der zusätzlich die Sequenzreihenfolge zur ursprünglichen Aminosäuresequenz umgekehrt wurde (retro = rückwärts).

Das erfindungsgemäße Polymer ist aus identischen Monomeren aufgebaut, oder enthält verschiedene Monomere.

In einer Alternative ist das erfindungsgemäße Polymer aus jeder beliebigen Kombination aus 2, 3, 4, 5, 6, 7, 8, 9, 10 oder mehr der oben beschriebenen Monomere aufgebaut.

In einer Ausführung ist das Polymer ein Dimer aus zwei D3-Monomeren (SEQ ID NO:13). In einer weiteren Ausführung ist das erfindungsgemäße Polymer ein Dimer aus zwei RD2-Monomeren (SEQ ID NO:76).

Dimere können beispielsweise über chemische Synthese bzw. Peptidsynthese hergestellt werden.

In einer Ausführung der Erfindung sind die Monomere kovalent miteinander verknüpft. In einer weiteren Ausführung sind die Monomere nicht kovalent miteinander verbunden.
Eine kovalente Verbindung bzw. Verknüpfung der Monomer-Einheiten liegt im Sinne der Erfindung vor, falls die Peptide Kopf an Kopf, Schwanz an Schwanz oder Kopf an Kopf linear miteinander verknüpft werden, ohne dass dazwischen Linker oder Linker-Gruppen eingesetzt werden.

Eine nicht kovalente Verknüpfung im Sinne der Erfindung liegt vor, falls die Monomere über Biotin und Streptavidin, insbesondere Streptavidin-Tetramer miteinander verknüpft sind.

In einer Variante der vorliegenden Erfindung können die Monomere linear miteinander verknüpft sein, insbesondere wie oben beschrieben. In einer anderen Variante sind die Monomere verzweigt miteinander zu dem erfindungsgemäßen Polymer verknüpft.

Bei einem verzweigten Polymer kann es sich erfindungsgemäß um ein Dendrimer handeln, bei dem die Monomere kovalent oder nicht kovalent miteinander verknüpft sind.

Alternativ können die Monomere auch mit einem Plattform-Molekül (wie z.B. PEG oder Zucker) verknüpft sein und so ein verzweigtes Polymer bilden.

Alternativ sind auch Kombinationen dieser Optionen möglich.

Das Polymer ist dadurch gekennzeichnet, dass es Amyloid-Beta-Oligomere mit einer Dissoziationskonstante von höchstens 1 µM, bevorzugt 800, 600, 400, 200, 100, 10 nM, besonders bevorzugt, 1.000, 900, 800, 700, 600, 500, 400, 300, 200, 100, 50 pM, insbesondere höchstens 20 pM bindet.

In einer weiteren Ausführungsform ist das erfindungsgemäße Polymer dadurch gekennzeichnet, dass es Amyloid-Beta-Oligomere mit einer Dissoziationskonstante von höchstens 500, 5 pM bindet.

Das erfindungsgemäße Polymer ist geeignet zur Verwendung in der Medizin.

In einer Ausführung handelt es sich um ein Polymer zur Behandlung von Morbus Alzheimer. In einer weiteren Ausführung handelt es sich um ein Polymer, welches zur Behandlung von Parkinson, CJD (*Creutzfeldt Jakob Disease*), oder Diabetes eingesetzt werden kann.

Die erfindungsgemäß aufgebauten Polymere aus Monomeren, die ihrerseits an A-Beta-Oligomere binden, zeigen deutliche, synergetische Effekte in Bezug auf ihre Selektivität und Affinität zu den A-Beta-Oligomeren im Vergleich zu den Monomeren. Mit anderen Worten: Die erfindungsgemäßen Polymere sind den Monomeren überlegen. Synergetische Effekte im Sinne der vorliegenden Erfindung sind Effekte, die eine höhere Selektivität und Affinität bezüglich der A-Beta-Oligomere zeigen, insbesondere des K_{D}-Wertes betreffend die Bindung an A-Beta-Oligomere als die Monomere einzeln oder in ihrer Addition.

Unter einem Linker sind ein oder mehrere Moleküle zu verstehen, die über kovalente Bindungen an die Monomere gebunden sind, wobei diese Linker untereinander auch durch kovalente Bindungen verknüpft sein können.
In einer Alternative der vorliegenden Erfindung werden durch die Linker die durch die Monomere vorgegebenen Eigenschaften des Polymers, nämlich die Bindung an A-Beta-Oligomere, nicht verändert.
In einer weiteren Alternative bewirken die Linker eine Veränderung der Eigenschaften des Polymers, die durch die Monomere vorgegeben sind. In einer solchen Ausführung wird die Selektivität und/oder Affinität der erfindungsgemäßen Polymere bezüglich der A-Beta-Oligomere verstärkt und/oder die Dissoziationskonstante herabgesetzt. In einer weiteren Ausführung werden die Linker so ausgewählt oder können so angeordnet sein, dass sie die sterische Wirkung der erfindungsgemäßen Polymere derart verändern, dass diese selektiv nur an A-Beta-Oligomere einer bestimmten Größe binden.

Ein nicht limitierendes Beispiel für einen Linker ist die Aminosäuresequenz "nwn".

Eine solche Änderung der sterischen Wirkung der erfindungsgemäßen Polymere kann auch durch den Aufbau verzweigter erfindungsgemäßen Polymere, durch Dendrimere eines besonderen Aufbaus oder den entsprechenden Aufbau des Polymers mittels Monomere und einem Plattformmolekül oder Kombinationen dieser Optionen erreicht werden.

Gegenstand der vorliegenden Erfindung ist auch eine Zusammensetzung enthaltend das erfindungsgemäße Polymer, insbesondere zur Behandlung von Morbus Alzheimer.

Offenbart ist ferner eine Zusammensetzung enthaltend das erfindungsgemäße Polymer, insbesondere zur Verhinderung von toxischen A-Beta-Oligomeren oder zur Zerstörung von daraus gebildeten Polymere oder Fibrillen.
Die erfindungsgemäße "Zusammensetzung" kann z. B. ein Impfstoff, ein Medikament (z. B. in Tablettenform), eine Injektionslösung, ein Nahrungs- oder Nahrungsergänzungsmittel sein enthaltend das erfindungsgemäße Polymer in einer aufgrund des Fachwissens herzustellenden Formulierung.

Die erfindungsgemäßen Polymere enttoxifizieren die A-Beta-Oligomere oder daraus gebildete Polymere sowie Fibrillen, indem sie daran binden und so in nicht toxische Verbindungen überführen. Substanzen, die in der Lage sind, die Bildung von Fibrillen zu inhibieren, müssen nicht notwendigerweise auch in der Lage sein, vorgeformte Fibrillen zu zerstören, da vorgeformte A-beta-Fibrillen sehr stabil sind und nur sehr schwer und langsam wieder zerstört werden können.
Ferner offenbart ist auch ein Verfahren zur Enttoxifizierung der A-Beta-Oligomeren, daraus gebildeten Polymeren oder Fibrillen

Aufgrund ihrer Eigenschaft sowohl die kleinen, frei diffundierbaren A-Beta-Oligomere, größere A-Beta-Oligomere bis hin zu Fibrillen zu binden, sind die erfindungsgemäßen Polymere in allen Stadien der AD einsetzbar. Auf Basis der Lehre der vorliegenden Erfindung können Polymere hergestellt werden, die auch selektiv an verschiedene Formen der A-Beta-Oligomere binden.

Ferner offenbart ist auch ein Verfahren zur Herstellung des erfindungsgemäßen Polymers. Bevorzugte Verfahren sind beispielsweise Peptid-Synthese-Verfahren (Peptide), die rekombinante Herstellung von Proteinen und anerkannte organische Synthese-Methoden für beliebige sog. *low-molecular-weight compounds.*

Die vorliegende Erfindung betrifft auch die Verwendung des Polymers als Sonde zur Identifizierung, qualitativen und/oder quantitativen Bestimmung von Amyloid-Beta-Oligomeren. Ferner offenbart ist auch eine Sonde, enthaltend das erfindungsgemäße Polymer zur Identifizierung, qualitativen und/oder quantitativen Bestimmung von Amyloid-Beta-Oligomeren.

Solche Sonden sind von großer Bedeutung, da damit eine frühe Diagnose der Alzheimerschen Demenz möglich wird. Damit kann der Krankheit schon in einem sehr frühen Stadium entgegengewirkt werden.
Solche molekularen Sonden enthalten das erfindungsgemäße Polymer und können den Patienten z.B. intravenös injiziert werden. Weitere Bestandteile der Sonden können sein: Farbstoffe, Fluoreszenzfarbstoffe, radioaktive Isotope (z.B. PET), Gadolinium (MRI) und/oder Bestandteile, die für Sonden in der Bildgebung eingesetzt werden. Nach Passage über die Bluthirnschranke können die Sonden an A-Beta-Oligomere und/oder Plaques binden. Die so markierten A-Beta-Oligomere und/oder Plaques können mittels bildgebender Verfahren wie z.B. SPECT, PET, CT, MRT, Protonen-MR-Spektroskopie usw. sichtbar gemacht werden.

Weiterer Gegenstand der vorliegenden Erfindung ist die Verwendung des Polymers zur Verhinderung der Vermehrung von toxischen Amyloid-Beta-Oligomeren.

Die erfindungsgemäßen Polymeren werden außerdem auch zur Bildung von nichttoxischen Polymer-Amyloid-Beta-Oligomer-Komplexen eingesetzt.

Ferner offenbart ist ein Kit, enthaltend das erfindungsgemäße Polymer. In einem solchen Kit können die erfindungsgemäßen Polymere in Behältern ggf. mit/in Puffern oder Lösungen verpackt sein. Alle Komponenten des Kits können in demselben Behälter oder getrennt voneinander verpackt sein. Ferner kann das Kit Anweisungen für dessen Gebrauch enthalten. Ein solches Kit kann beispielsweise die erfindungsgemäßen in einer Injektionsflasche mit Stopfen und/oder Septum enthalten. Ferner kann darin beispielsweise auch eine Einmal-Spritze enthalten sein.

Sequenzen, die erfindungsgemäß einsetzbar sind, sind nachfolgend dargestellt:
RD 2: ptlhthnrrrrr (SEQ ID NO: 1)
D3D3: rprtrlhthrnrrprtrlhthrnr (SEQ ID NO:13)
D3nwnD3: rprtrlhthrnrnwnrprtrlhthrnr (SEQ ID NO:14)
doppel-D3-freie-Ntermini: (rprtrlhthrnr)₂-PEG3 (SEQ ID NO:15)
doppel-D3-freie-Ctermini: PEG5-(rprtrlhthrnr)₂ (SEQ ID NO:16)
kqhhveygsdhrfead (SEQ ID NO:2)
shyrhisp (SEQ ID NO:3)
giswqqshhlva (SEQ ID NO:4)
prtrlhth (SEQ ID NO:5)
qshyrhispaqv (SEQ ID NO:6)
qshyrhispdqv (SEQ ID NO:7)
qshyrhispar (SEQ ID NO:8)
kshyrhispakv (SEQ ID NO:9)
rprtrlhthrnr (SEQ ID NO:10)
rprtrlhthrte (SEQ ID NO:11)
kprtrlhthrnr (SEQ ID NO:12)
daefrhdsgye (SEQ ID NO:17)
hhghspnvsqvr (SEQ ID NO:18)
gsfstqvgslhr (SEQ ID NO:19)
htgtqsyvprl (SEQ ID NO:20)
tlayaraymvap (SEQ ID NO:21)
tlayaraymvap (SEQ ID NO:22)
atpqndlktfph (SEQ ID NO:23)
tqpetdllrvqf (SEQ ID NO:24)
citwpptglty (SEQ ID NO:25)
tfletgpiyadg (SEQ ID NO:26)
lvppthrhwpvt (SEQ ID NO:27)
appgnwrnylmp (SEQ ID NO:28)
dnysnyvpgtkp (SEQ ID NO:29)
svsvgmkpsprp (SEQ ID NO:30)
slpnpfsvssfg (SEQ ID NO:31)
yvhnpyhlpnpp (SEQ ID NO:32)
crrlhtyigpvt (SEQ ID NO:33)
gatmkkmddhtv (SEQ ID NO:34)
lgktqklsdahs (SEQ ID NO:35)
ddqarpymaygp (SEQ ID NO:36)
gdtwvnmvsmvh (SEQ ID NO:37)
gytwvnmvsmvh (SEQ ID NO:38)
wtntvarlatpy (SEQ ID NO:39)
qtqalyhsrqvh (SEQ ID NO:40)
nsqtqtlhlfph (SEQ ID NO:41)
hntsanilhssh (SEQ ID NO:42)
shinptsfwpap (SEQ ID NO:43)
tfsnplymwprp (SEQ ID NO:44)
gpspfnpqptpv (SEQ ID NO:45)
fsdhksptpppr (SEQ ID NO:46)
stsvyppppsaw (SEQ ID NO:47)
yglptqansmql (SEQ ID NO:48)
hnrtdntyirpt (SEQ ID NO:49)
lqqplgnnrpns (SEQ ID NO:50)
kpedsaaypqnr (SEQ ID NO:51)
rpedsvitktqnt (SEQ ID NO:52)
raadsgctptkh (SEQ ID NO:53)
rprtrlhthrnt (SEQ ID NO:54)
rprtrlhthtnv (SEQ ID NO:55)
rprtrlhthtnr (SEQ ID NO:56)
rprtrlhthrkq (SEQ ID NO:57)
rprtrlhtlrnr (SEQ ID NO:58)
rrrsplhthrnr (SEQ ID NO:59)
lrsprqrripri (SEQ ID NO:60)
rkrqlrmttprp (SEQ ID NO:61)
shyrhispaqk (SEQ ID NO:62)
doppel-D3-freie-Ntermini: (rprtrlhthrnr)2- (SEQ ID NO:63)
doppel-D3-freie-Ctermini: (rprtrlhthrnr)2 (SEQ ID NO:64)
DB 3: rpitrlrthqnr (SEQ ID NO: 65),
D3: rprtrlhthrnr (SEQ ID NO:66)
RD 1: pnhhrrrrrrtl (SEQ ID NO: 67)
RD 3: rrptlrhthnrr (SEQ ID NO: 68)
D3-delta-hth: rprtrlrnr (SEQ ID NO:69)
NT-D3: rprtrl (SEQ ID NO: 70)
DB 1: rpitrlhtnrnr (SEQ ID NO: 71),
DB 2:rpittlqthqnr (SEQ ID NO: 72),
DB 4: rprtrlrthqnr (SEQ ID NO: 73 ()
DB 5: rpitrlqtheqr (SEQ ID NO. 74)
D3-delta-hth D3-delta-hth: rprtrlrnrrprtrlrnr (SEQ ID NO:75)
RD 2- RD 2: ptlhthnrrrrrptlhthnrrrrr (SEQ ID NO: 76)
DO 3: sgwhynwqywwk (SEQ ID NO:77)
rprtrsgwhynwqywwkrnr (SEQ ID NO:78)
ptlsgwhynwqywwkrrrrr (SEQ ID NO:79)

Antikörper, die an A-Beta binden sind nachfolgend dargestellt:
Antikörper, die
a) an eine retro-inverse Sequenz des Amyloid beta-Peptids oder Amyloid beta-Peptid-Teilfragmenten binden und/oder
b) an die Multimerisierungsdomäne des Amyloid beta-Peptids binden und auch an das Amyloid beta-Peptid und/oder
c) an eine der oben genannten erfindungsgemäßen Sequenzen ausgewählt aus der Gruppe:
   SEQ ID NO:1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12, SEQ ID NO:13, SEQ ID NO:14, SEQ ID NO:15, SEQ ID NO:16, SEQ ID NO:17, SEQ ID NO:18, SEQ ID NO:19, SEQ ID NO:20, SEQ ID NO:21, SEQ ID NO:22, SEQ ID NO:23, SEQ ID NO:24, SEQ ID NO:25, SEQ ID NO:26, SEQ ID NO:27, SEQ ID NO:28, SEQ ID NO:29, SEQ ID NO:30, SEQ ID NO:31, SEQ ID NO:32, SEQ ID NO:33, SEQ ID NO:34, SEQ ID NO:35, SEQ ID NO:36, SEQ ID NO:37, SEQ ID NO:38, SEQ ID NO:39, SEQ ID NO:40, SEQ ID NO:41, SEQ ID NO:42, SEQ ID NO:43, SEQ ID NO:44, SEQ ID NO:45, SEQ ID NO:46, SEQ ID NO:47, SEQ ID NO:48, SEQ ID NO:49, SEQ ID NO:50, SEQ ID NO:51, SEQ ID NO:52, SEQ ID NO:53, SEQ ID NO:54, SEQ ID NO:55, SEQ ID NO:56, SEQ ID NO:57, SEQ ID NO:58, SEQ ID NO:59, SEQ ID NO:60, SEQ ID NO:61, SEQ ID NO:62, SEQ ID NO:63, SEQ ID NO:64. SEQ ID NO:65, SEQ ID NO:66, SEQ ID NO:67, SEQ ID NO:68, SEQ ID NO:69, SEQ ID NO:70, SEQ ID NO:71, SEQ ID NO:72, SEQ ID NO:73, SEQ ID NO:74, SEQ ID NO:75, SEQ ID NO:76, SEQ ID NO:77, SEQ ID NO:78 und SEQ ID NO:79 oder homologe Sequenzen davon;
   binden.

### Beispiele:

### Beispiel 1:

Es wurden folgende vier D3-Dimere hergestellt:
D3D3: rprtrlhthrnrrprtrlhthrnr (SEQ ID NO:13)
D3nwnD3: rprtrlhthrnrnwnrprtrlhthrnr (SEQ ID NO:14)
doppel-D3-freie-Ntermini: (rprtrlhthrnr)₂-PEG3 (SEQ ID NO:15)
doppel-D3-freie-Ctermini: PEG5-(rprtrlhthrnr)₂ (SEQ ID NO:16)

Weiterhin wurden Dimere (über chemische Synthese bzw. Peptidsynthese) hergestellt, die aus D3 und D1 (Sequenz qshyrhispaqv, SEQ ID NO:6) bestehen oder D3 und V1, wobei V1 einer Variante des D1 mit Deletion der ersten Aminosäure am N-Terminus (Sequenz shyrhispaqk, SEQ ID NO:62)
D3D1
D3V1
V1D3

### Beispiel 2:

### Thioflavin T-Test

Die im Beispiel 1 hergestellten D3-Dimere sowie ein D3-Monomer wurden in einem üblichen, dem Fachmann bekannten Thioflavin-T-(ThT)-Test verglichen. ThT ist ein Farbstoff, der bei Bindung an reguläre Fibrillen eine höhere Fluoreszenz besitzt und somit als Maß für die Fibrillierung dient. Die D3-Dimere reduzierten die A-beta-Fibrillierung viel effizienter als das D3-Monomer.

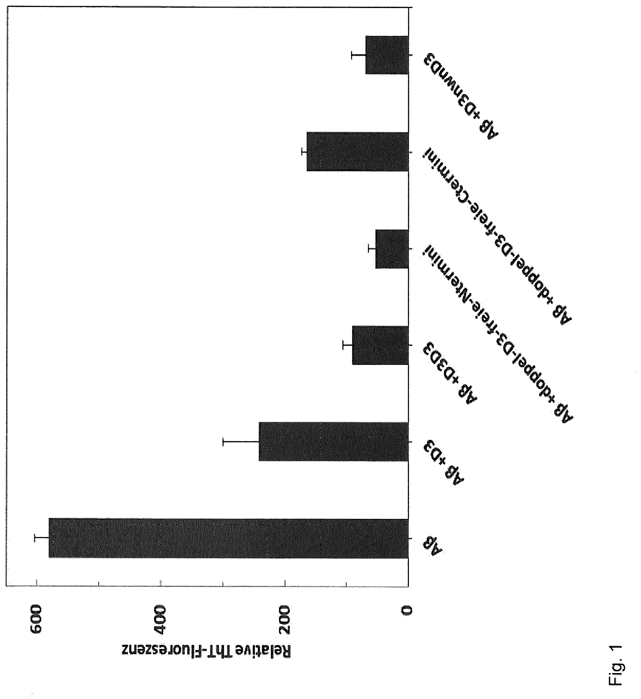

Die Figur 1 zeigt die Ergebnisse des ThT-Tests zur Analyse des Aggregationsverhaltens von A-Beta 1-42 in Anwesenheit von D3 bzw. D3-Multimeren. A-Beta-Pellets wurde durch Vorinkubation mit HFIP über Nacht und anschließender Evaporation mittels Vakuumzentrifuge hergestellt (222 µM Aß in HFIP). A-beta-Pellets wurden in 250 µL PBS pH 7,4 (inkl. 10 µM ThT und 10 µM D3 bzw. D3-Multimer) resuspendiert und für eine 4-fach-Bestimmung aliquotiert. Die Fluoreszenz des ThT wurde bei λex 440 nm und λem = 490 nm im Fluoreszenzspektrometer gemessen.

### Beispiel 3:

Die im Beispiel 1 hergestellten D3D1-Dimere sowie die D1- und D3-Monomere wurden in einem üblichen, dem Fachmann bekannten ThT-Test ChTThT-Test verglichen.

Die D3D1-Dimere waren reduzierten die A-beta-Fibrillierung viel effizienter als das D3-Monomer.

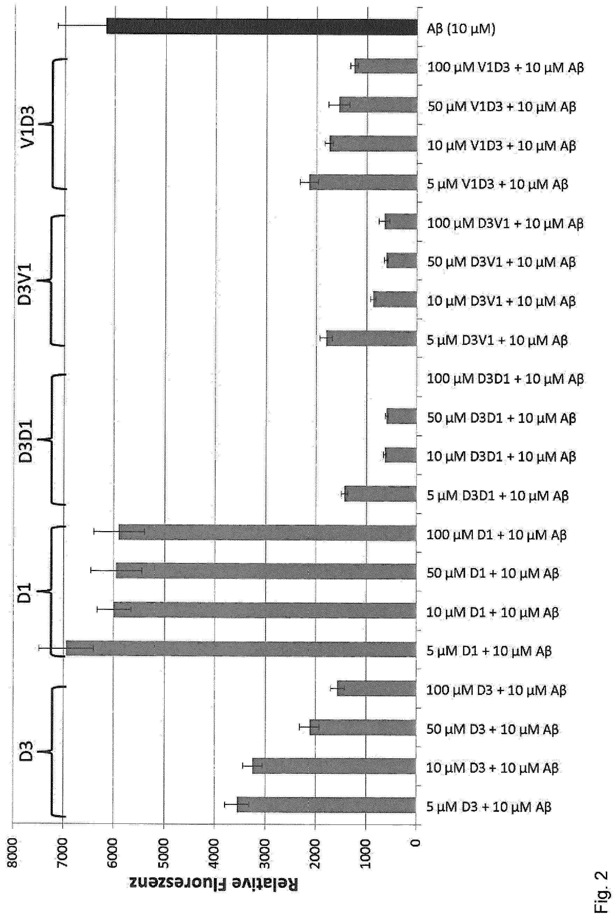

Die Figur 2 zeigt die Ergebnisse des ThT-Tests zur Analyse des Aggregationsverhaltens von A-beta1-42 in Anwesenheit von D3 und D1 bzw. D3D1-Multimeren. A-beta wurde in HFIP über Nacht vorinkubiert (222 µM A-beta in HFIP). Anschließend wurden Aliquots à 13.5 µg hergestellt und das HFIP über Nacht abgedampft. A-beta-Pellets wurden in 270 uL PBS pH 7,4 (inkl. 10 µM ThT) resuspendiert und anschließend die entsprechende Konzentration D3 bzw D3D1-Multimer (5 µM, 10 µM, 50 µM bzw 100 µM) zugegeben, danach wurde für eine 5-fach-Bestimmung aliquotiert. Die Fluoreszenz des ThT wurde bei λex 440 nm und λem = 490 nm im Fluoreszenzspektrometer gemessen.

### Beispiel 4:

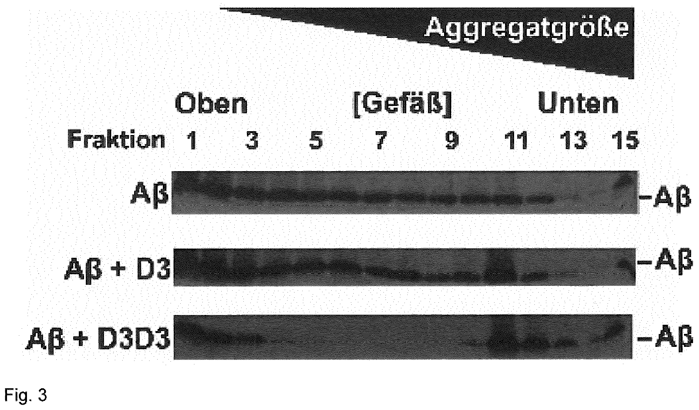

Die im Beispiel 1 hergestellten D3-Dimere sowie das D3-Monomer wurden mit Abeta1-42 vermischt und einer Dichtegradientenzentrifugation unterworfen. Die Größenverteilungen von A-beta in Lösung und A-beta-Peptid-Mischungen wurden dabei durch Sedimentationsanalyse auf einem Iodixanol-Gradienten (5-50 %) untersucht. Die Mischungen enthielten 80 µM A-beta und 20 (D3) oder 10 (D3D3) µM Peptid. Nach der Zentrifugation wurden 15 Fraktionen von je 140 µl durch sequentielles Pipettieren von der Oberfläche gewonnen und mittels denaturierender Polyacrylamidgelelektrophorese (SDS-PAGE) und anschließender Silberfärbung analysiert. Die Ergebnisse (Fig. 3) zeigen, dass die Peptide den Gehalt an A-beta-Oligomeren signifikant reduzieren (Fraktionen 4-10), D3D3 zu dem untersuchten Zeitpunkt in einem weitaus höheren Maße als D3. Es entstehen große Aggregate, die in den Fraktionen 12-15 detektierbar sind. In weiteren Studien stellte sich heraus, dass D3-A-beta-Oligomere präzipitiert und in große, amorphe und untoxische Aggregate umwandelt (Funke et al., ACS Chem. Neurosci. 2010).

### Beispiel 5:

Erfindungsgemäße Substanzen sind in der Lage, die Bildung von Fibrillen zu inhibieren und vorgeformte Fibrillen zu zerstören. Es wurde untersucht wie effizient Inhibitor-Dimere (D3D3 SEQ ID NO: 13, RD2RD2 SEQ ID NO: 76, D3(Δ)delta-hthD3(Δ)delta-hth SEQ ID NO: 75) im Vergleich zu den entsprechenden Inhibitor-Monomeren (D3 SEQ ID NO: 66, RD2 SEQ ID NO: 1, D3(Δ)delta-hth SEQ ID NO: 69) in einem Thioflavin T (ThT)-Disfibrilisationsassay sind.

Dabei wird untersucht, welcher Anteil an vorgeformten ThT-positiven Fibrillen nach einer bestimmten Inkubationszeit reduziert wird. Thioflavin T (ThT) verändert bei der Interaktion mit amyloiden, ThT-positiven Fibrillen seine spektroskopischen Eigenschaften messbar. Die Abnahme der gemessenen ThT Fluoreszenz (λem: 450 nm, λex: 490 nm) spiegelt eine Abnahme von β-Faltblatt-Strukturen wieder, die charakteristisch für Abeta-Fibrillen sind.

Zur Herstellung von vorgeformten A-beta-Fibrillen wurde 33 µM synthetisches Abeta(1-42) 6 Tage lang in 10 mM Natriumphosphat pH 7.4 und 8,5 % DMSO bei 37 °C inkubiert. Die dadurch entstandenen Abeta-Fibrillen wurden zu 20 µM mit 0,1 µg/µl der jeweiligen Substanz (D3D3, RD2RD2, D3delta-hthD3delta-hth, D3, RD2, D3Δhth) und 10 µM ThT zusammengegeben. 50 µL jedes Reaktionsansatzes wurden in eine Vertiefung einer schwarzen Mikrotiterplatte mit 384 Vertiefungen (Nunc, Langenselbold) gefüllt. Die ThT-Fluoreszenz wurde über 17,5 h verfolgt. Dabei wurde die Mikrotiterplatte vor jeder Messung 30 s geschüttelt. Alle Proben wurden fünffach gemessen.

Bei der Auswertung wurden Mittelwert, Standartabweichung und Signifikanz (zwischen monomeren und dimeren Effektoren) ermittelt. Die ThT-Fluoreszenz der Effektoren wurde auf die ThT-Fluoreszenz von Abeta-Fibrillen in Abwesenheit von Inhibitor-Substanzen normiert.

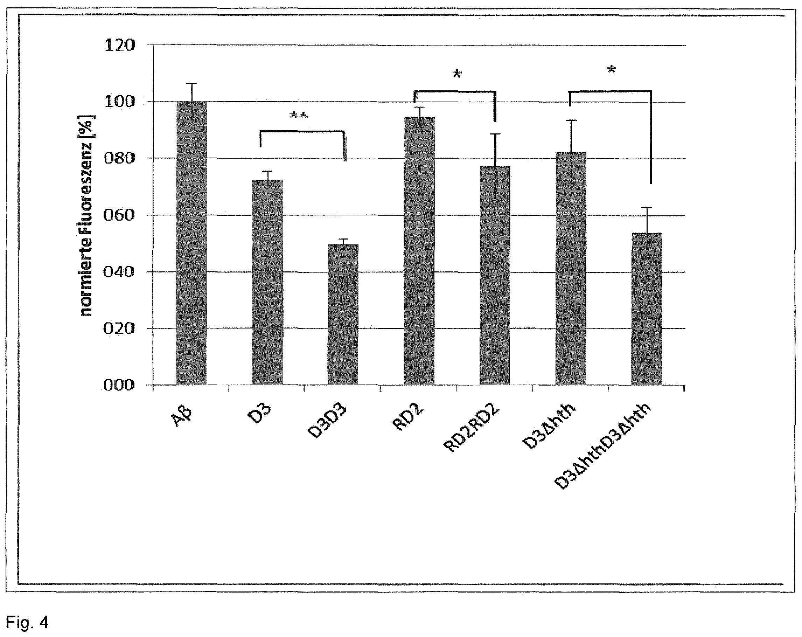

Alle getesteten Inhibitor-Substanzen zeigten eine Abnahme der ThT-Fluoreszenz (Fig. 4). Dies zeigt, dass sie alle in der Lage waren vorgeformte A-beta-Fibrillen in nicht-fibrilläre A-beta-Spezies umzuwandeln. Die dimeren Substanzen D3D3, RD2RD2 und D3delta-hthD3delta-hth zeigten dabei einen signifikant stärkeren Effekt gegenüber den monomeren Peptiden D3, RD2 und D3delta-Δhth. Die Signifikanz wurde mittels Mann-Whitney Test ermittelt und ist in der Abbildung mit Sternchen angegeben: *, p < 0,05; **, p < 0,01.

### Beispiel 6:

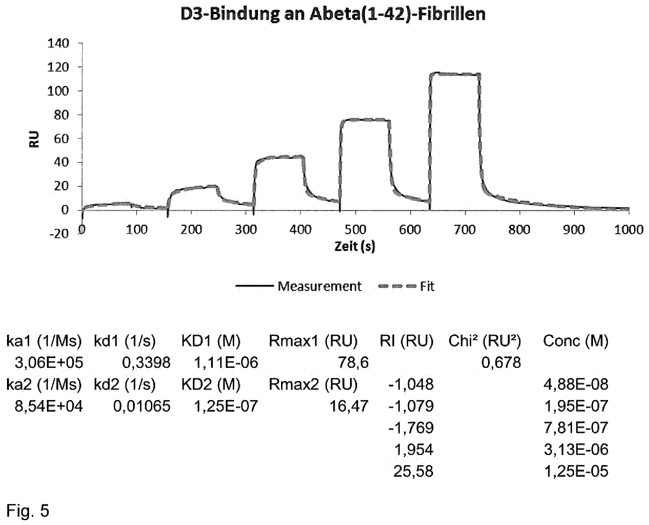

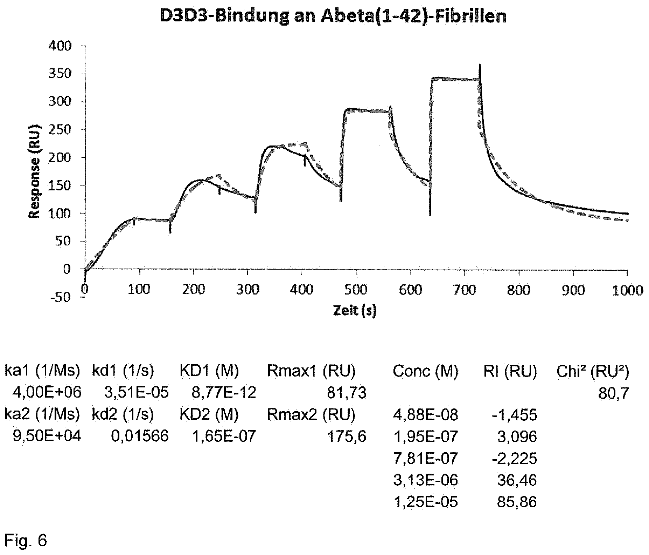

Um vergleichende quantitative Affinitätsdaten für D3 und D3D3 zu erhalten, wurden Abeta-1-42-Fibrillen präpariert, wie in Beispiel 6 beschrieben. Im Unterschied dazu wurde eine Mischung aus 90% Abeta-1-42 und 10 % Abeta-1-42, das aminoterminal biotinyliert war, für den Fibrillierungsversuch verwendet. Um diese Fibrillen von möglichen Anteilen anderer Abeta-Konformere, z.B. Monomere und amorphen Oligomere zu trennen, wurde die Fibrillen-Präparationen einem Dichtegradientenzentrifugationslauf unterworfen, wie in Beispiel 4 beschrieben. Nur die in den Fraktionen 11 und 12 erhaltenen Fibrillen wurden anschließend als Ligand in einer Oberflächenplasmonenresonanz-Analyse eingesetzt. Dabei wurden diese auf einem Streptavidin-beschichteten Chip (Sensor Chip SA) immobilisiert. Anschließend wurden die Affinitäten der Analyten D3 und D3D3 mittels "single cycle kinetic"-Analyse bestimmt. Die eingesetzten Analyt-Konzentrationen, die erhaltenen Sensorgramme (dicke Linien), die erhaltenen Fit-Kurven (dünne gestrichelte Linien) und die erhalten Dissoziationskonstanten sind in den Fig. 5 und 6 enthalten.

Aus den Ergebnissen wird klar, dass das D3-Monomer an zwei leicht unterschiedliche Bindungsstellen mit Dissoziationskonstanten von 1,1 µM und 0,12 µM bindet. Auch das D3-Dimer bindet an zwei unterschiedliche Bindungsstellen, eine davon mit einer zum D3-Monomer ähnlichen Dissoziationskonstanten von 0,17 µM, die andere aber mit einer um mehrere Größenordnungen geringeren Dissoziationskonstanten von 8,8 pM. Dies zeigt, dass polyvalente Substanzen (hier D3D3) fast 10000-fach stärker an ihr Ziel binden als die entsprechende monovalente Substanz (hier D3).

## Patentansprüche

1. Polymer, enthaltend mindestens zwei Substanzen (Monomere), die an Amyloid-Beta-Oligomere binden, wobei eine Substanz (Monomer) der mindestens zwei Substanzen SEQ ID NO: 1 (RD2) ist.

2. Polymer nach einem der vorangehenden Ansprüche **dadurch gekennzeichnet, dass** jedes Monomer, aus denen das Polymer aufgebaut ist, an ein Monomer und/oder Oligomer und/oder Fibrillen des Amyloid-Beta-Peptids mit einer Dissoziationskonstante (KD-Wert) von höchstens 500 pM bindet.

3. Polymer nach einem der vorangegangenen Ansprüche **dadurch gekennzeichnet, dass** es 2, 3, 4, 5, 6, 7, 8, 9, 10 oder mehr Monomere enthält.

4. Polymer nach einem der vorangehenden Ansprüche, dass weitere Monomere ausgewählt werden aus der Gruppe bestehend aus:
SEQ ID NO:1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12, SEQ ID NO:13, SEQ ID NO:14, SEQ ID NO:15, SEQ ID NO:16, SEQ ID NO:17, SEQ ID NO:18, SEQ ID NO:19, SEQ ID NO:20, SEQ ID NO:21, SEQ ID NO:22, SEQ ID NO:23, SEQ ID NO:24, SEQ ID NO:25, SEQ ID NO:26, SEQ ID NO:27, SEQ ID NO:28, SEQ ID NO:29, SEQ ID NO:30, SEQ ID NO:31, SEQ ID NO:32, SEQ ID NO:33, SEQ ID NO:34, SEQ ID NO:35, SEQ ID NO:36,SEQ ID NO:37, SEQ ID NO:38, SEQ ID NO:39, SEQ ID NO:40, SEQ ID NO:41, SEQ ID NO:42, SEQ ID NO:43, SEQ ID NO:44, SEQ ID NO:45, SEQ ID NO:46, SEQ ID NO:47, SEQ ID NO:48, SEQ ID NO:49, SEQ ID NO:50, SEQ ID NO:51, SEQ ID NO:52, SEQ ID NO:53, SEQ ID NO:54, SEQ ID NO:55, SEQ ID NO:56, SEQ ID NO:57, SEQ ID NO:58, SEQ ID NO:59, SEQ ID NO:60, SEQ ID NO:61, SEQ ID NO:62, SEQ ID NO:63, SEQ ID NO:64. SEQ ID NO:65, SEQ ID NO:66, SEQ ID NO:67, SEQ ID NO:68, SEQ ID NO:69, SEQ ID NO:70, SEQ ID NO:71, SEQ ID NO:72, SEQ ID NO:73, SEQ ID NO:74, SEQ ID NO:75, SEQ ID NO:76, SEQ ID NO:77, SEQ ID NO:78 und SEQ ID NO:79
oder jede beliebige Kombination aus 2, 3, 4, 5, 6, 7, 8, 9, 10 oder mehr dieser Monomere.

5. Polymer nach einem der vorangehenden Ansprüche **dadurch gekennzeichnet, dass** die Monomere ohne Linker, also unmittelbar miteinander verknüpft, oder mit einer Linker-Gruppe miteinander verknüpft sind.

6. Polymer nach einem der vorangehenden Ansprüche **dadurch gekennzeichnet, dass** die Monomere linear oder verzweigt miteinander verknüpft sind.

7. Polymer nach einem der vorangehenden Ansprüche **dadurch gekennzeichnet, dass** es sich um ein Dendrimer handelt oder dass die Monomere mit einem Platform-Molekül verknüpft sind oder in einer Kombination dieser Optionen verknüpft sind.

8. Polymer nach einem der vorangehenden Ansprüche **dadurch gekennzeichnet, dass** es Amyloid-Beta-Oligomere mit einer Dissoziationskonstante von höchstens 5 pM bindet.

9. Polymer nach einem der vorangehenden Ansprüche zur Verwendung in der Medizin.

10. Kit enthaltend ein Polymer nach einem der Ansprüche 1 - 8.

11. Sonde enthaltend ein Polymer nach einem der Ansprüche 1 -8.

12. Zusammensetzung, enthaltend ein Polymer nach einem der Ansprüche 1 - 8.

13. Verwendung des Polymers nach einem der Ansprüche 1-8 in vitro als Sonde zur Identifizierung, qualitativen und/oder quantitativen Bestimmung von Amyloid-Beta-Oligomeren.

14. Polymer nach einem der Ansprüche 1 -8 zur Verwendung in vivo zur Verhinderung von Amyloid-Beta-Oligomeren.

15. Polymere nach einem der Ansprüche 1 - 8 zur Verwendung in vivo zur Bildung von nicht toxischen Polymer-Amyloid-Beta-Oligomer-Komplexen.

## Claims

1. A polymer comprising at least two substances (monomers) which bind to amyloid-beta oligomers, where one substance (monomer) of the at least two substances is SEQ ID NO:1 (RD2).

2. The polymer as claimed in either of the preceding claims, **characterized in that** each monomer of which the polymer is composed, binds to a monomer and/or oligomer and/or fibrils of the amyloid-beta peptide with a dissociation constant (K_{D} value) of not more than 500 pm.

3. The polymer as claimed in any of the preceding claims, **characterized in that** it comprises 2, 3, 4, 5, 6, 7, 8, 9, 10 or more monomers.

4. The polymer as claimed in any of the preceding claims, **characterized in that** the monomers are selected from the group consisting of:
SEQ ID NO:1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12, SEQ ID NO:13, SEQ ID NO:14, SEQ ID NO:15, SEQ ID NO:16, SEQ ID NO:17, SEQ ID NO:18, SEQ ID NO:19, SEQ ID NO:20, SEQ ID NO:21, SEQ ID NO:22, SEQ ID NO:23, SEQ ID NO:24, SEQ ID NO:25, SEQ ID NO:26, SEQ ID NO:27, SEQ ID NO:28, SEQ ID NO:29, SEQ ID NO:30, SEQ ID NO:31, SEQ ID NO:32, SEQ ID NO:33, SEQ ID NO:34, SEQ ID NO:35, SEQ ID NO:36, SEQ ID NO:37, SEQ ID NO:38, SEQ ID NO:39, SEQ ID NO:40, SEQ ID NO:41, SEQ ID NO:42, SEQ ID NO:43, SEQ ID NO:44, SEQ ID NO:45, SEQ ID NO:46, SEQ ID NO:47, SEQ ID NO:48, SEQ ID NO:49, SEQ ID NO:50, SEQ ID NO:51, SEQ ID NO:52, SEQ ID NO:53, SEQ ID NO:54, SEQ ID NO:55, SEQ ID NO:56, SEQ ID NO:57, SEQ ID NO:58, SEQ ID NO:59, SEQ ID NO:60, SEQ ID NO:61, SEQ ID NO:62, SEQ ID NO:63, SEQ ID NO:64. SEQ ID NO:65, SEQ ID NO:66, SEQ ID NO:67, SEQ ID NO:68, SEQ ID NO:69, SEQ ID NO:70, SEQ ID NO:71, SEQ ID NO:72, SEQ ID NO:73, SEQ ID NO:74, SEQ ID NO:75, SEQ ID NO:76, SEQ ID NO:77, SEQ ID NO:78 and SEQ ID NO:79
or any combination of 2, 3, 4, 5, 6, 7, 8, 9, 10 or more of the above-described monomers.

5. The polymer as claimed in any of the preceding claims, **characterized in that** the monomers are linked to each other without linker, that is to say directly, or linked to each other via a linker group.

6. The polymer as claimed in any of the preceding claims, **characterized in that** the monomers are linked to each other in a linear or branched fashion.

7. The polymer as claimed in any of the preceding claims, **characterized in that** it is a dendrimer or **in that** the monomers are linked to a platform molecule or linked in a combination of these options.

8. A polymer, **characterized in that** it binds amyloid-beta oligomers with a dissociation constant of not more than 5 pm.

9. The polymer as claimed in any of the preceding claims for use in medicine.

10. A kit comprising a polymer as claimed in any of claims 1 - 8.

11. A probe comprising a polymer as claimed in any of claims 1 - 8.

12. A composition comprising a polymer as claimed in any of claims 1 - 8.

13. The use in vitro of he polymer as claimed in any of claims 1 - 8 as a probe for the identification, qualitative and/or quantitative determination of amyloid-beta oligomers.

14. The polymer as claimed in any of claims 1 - 8 for the use in vivo for preventing amyloid-beta oligomers.

15. The polymer as claimed in any of claims 1 - 8 for the use in vivo for the formation of nontoxic polymer-amyloid-beta oligomer complexes.

## Revendications

1. Un polymère contenant au moins deux substances (monomères) qui lient aux oligomères bêta- amyloïde , où une substance (monomère) des au moins deux substances est SEQ ID NO:1 (RD2).

2. Le polymère selon l'une quelconque des revendications précédentes, **caractérisée en ce que** chaque monomère, desquels le polymère est composé, lie à un monomère et/ou oligomère et/ou aux fibrilles du peptide bêta-amyloïde avec une dissociation constante (K_{D} valeur) de 500 pm au maximum.

3. Le polymère selon l'une quelconque des revendications précédentes, **caractérisée en ce qu'**il contient 2, 3, 4, 5, 6, 7, 8, 9, 10 des monomères ou plus.

4. Le polymère selon l'une quelconque des revendications précédentes, **caractérisée en ce que** des monomères sont selectionnés du groupe consistant en:
SEQ ID NO:1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11. SEQ ID NO:12, SEQ ID NO:13, SEQ ID NO:14, SEQ ID NO:15, SEQ ID NO:16, SEQ ID NO:17, SEQ ID NO:18, SEQ ID NO:19, SEQ ID NO:20, SEQ ID NO:21, SEQ ID NO:22, SEQ ID NO:23, SEQ ID NO:24, SEQ ID NO:25, SEQ ID NO:26, SEQ ID NO:27, SEQ ID NO:28, SEQ ID NO:29, SEQ ID NO:30, SEQ ID NO:31, SEQ ID NO:32, SEQ ID NO:33, SEQ ID NO:34, SEQ ID NO:35, SEQ ID NO:36, SEQ ID NO:37, SEQ ID NO:38, SEQ ID NO:39, SEQ ID NO:40, SEQ ID NO:41, SEQ ID NO:42, SEQ ID NO:43, SEQ ID NO:44, SEQ ID NO:45, SEQ ID NO:46, SEQ ID NO:47, SEQ ID NO:48, SEQ ID NO:49, SEQ ID NO:50, SEQ ID NO:51, SEQ ID NO:52, SEQ ID NO:53, SEQ ID NO:54, SEQ ID NO:55, SEQ ID NO:56, SEQ ID NO:57, SEQ ID NO:58, SEQ ID NO:59, SEQ ID NO:60, SEQ ID NO:61, SEQ ID NO:62, SEQ ID NO:63, SEQ ID NO:64. SEQ ID NO:65, SEQ ID NO:66, SEQ ID NO:67, SEQ ID NO:68, SEQ ID NO:69, SEQ ID NO:70, SEQ ID NO:71, SEQ ID NO:72, SEQ ID NO:73, SEQ ID NO:74, SEQ ID NO:75, SEQ ID NO:76, SEQ ID NO:77, SEQ ID NO:78 et SEQ ID NO:79
ou n'importe quelle combination de 2, 3, 4, 5, 6, 7, 8, 9, 10 ou plus de ces monomères.

5. Le polymère selon l'une quelconque des revendications précédentes, characterisée en ce que les monomères sont liés l'un avec l'autre sans liant, ca veut dire directement, ou sont liés l'un avec l'autre par un groupe des liants.

6. Le polymère selon l'une quelconque des revendications précédentes, **caractérisée en ce que** les monomères sont liés l'un avec l'autre dans une manière linéaire ou ramifiée.

7. Le polymère selon l'une quelconque des revendications précédentes, **caractérisée en ce qu'**il s'agit d'un dendrimère ou **en ce que** les monomères sont liés avec un molécule de plate-forme ou sont liés ensemble dans une combination dedites options.

8. Le polymère selon l'une quelconque des revendications précédentes, **caractérisée en ce qu'**il lie des oligomères bêta- amyloïde avec une constante de dissociation de de 5 pm au maximum.

9. Le polymère selon l'une quelconque des revendications précédentes pour l'utilisation en médicine.

10. Un kit contenant un polymère selon l'une quelconque des revendications 1 - 8.

11. Une sonde contenant un polymère selon l'une quelconque des revendications 1 - 8.

12. Une composition contenant un polymère selon l'une quelconque des revendications 1 - 8.

13. L'utilisation in vitro du polymère selon l'une quelconque des revendications 1 - 8 comme sonde pour l'identification, détermination qualitative et/ou quantitative des oligomères bêta amyloïde.

14. Le polymère selon l'une quelconque des revendications 1 - 8 pour l'utilisation in vivo comme sonde pour la prévention des oligomères bêta amyloïde.

15. Le polymère selon l'une quelconque des revendications 1 - 8 pour l'utilisation in vivo pour la formation des complexes de polymère-oligomère-bêta amyloïde non toxiques.


### Sequence Listing

<110> Forschungszentrum Juelich GmbH
<120> Polymere, enthaltend multivalente Amyloid-Beta-bindende D-Peptide und deren Verwendung
<130> FZJ1201DE
<160> 79
<170> patentin version 3.5
<210> 1
   <211> 12
   <212> PRT
   <213> Artificial Sequence
<220>
   <223> D-Peptid
<400> 1
   Pro Thr Leu His Thr His Asn Arg Arg Arg Arg Arg
   1               5                   10
<210> 2
   <211> 16
   <212> PRT
   <213> artificial sequence
<220>
   <223> D-Peptid
<400> 2
   Lys Gln His His Val Glu Tyr Gly Ser Asp His Arg Phe Glu Ala Asp
   1               5                   10                  15
<210> 3
   <211> 8
   <212> PRT
   <213> artificial sequence
<220><223> D-Peptid
<400> 3
   Ser His Tyr Arg His Ile Ser Pro
   1               5
<210> 4
   <211> 12
   <212> PRT
   <213> artificial sequence
<220><223> D-Peptid
<400> 4
   Gly Ile Ser Trp Gln Gln Ser His His Leu Val Ala
   1               5                   10
<210> 5
   <211> 8
   <212> PRT
   <213> artificial sequence
<220><223> D-Peptid
<400> 5
   Pro Arg Thr Arg Leu His Thr His
   1               5
<210> 6
   <211> 12
   <212> PRT
   <213> artificial sequence
<220><223> D-Peptid
<400> 6
   Gln Ser His Tyr Arg His Ile Ser Pro Ala Gln Val
   1               5                   10
<210> 7
   <211> 12
   <212> PRT
   <213> artificial sequence
<220><223> D-Peptid
<400> 7
   Gln Ser His Tyr Arg His Ile Ser Pro Asp Gln Val
   1               5                   10
<210> 8
   <211> 11
   <212> PRT
   <213> artificial sequence
<220><223> D-Peptid
<400> 8
   Gln Ser His Tyr Arg His Ile Ser Pro Ala Arg
   1               5                   10
<210> 9
   <211> 12
   <212> PRT
   <213> artificial sequence
<220><223> D-Peptid
<400> 9
   Lys Ser His Tyr Arg His Ile Ser Pro Ala Lys Val
   1               5                   10
<210> 10
   <211> 12
   <212> PRT
   <213> artificial sequence
<220><223> D-Peptid
<400> 10
   Arg Pro Arg Thr Arg Leu His Thr His Arg Asn Arg
   1               5                   10
<210> 11
   <211> 12
   <212> PRT
   <213> artificial sequence
<220><223> D-Peptid
<400> 11
   Arg Pro Arg Thr Arg Leu His Thr His Arg Thr Glu
   1               5                   10
<210> 12
   <211> 12
   <212> PRT
   <213> artificial sequence
<220><223> D-Peptid
<400> 12
   Lys Pro Arg Thr Arg Leu His Thr His Arg Asn Arg
   1               5                   10
<210> 13
   <211> 24
   <212> PRT
   <213> artificial sequence
<220><223> D-Peptid
<400> 13
   Arg Pro Arg Thr Arg Leu His Thr His Arg Asn Arg Arg Pro Arg Thr
   1               5                   10                  15
   Arg Leu His Thr His Arg Asn Arg
               20
<210> 14
   <211> 27
   <212> PRT
   <213> artificial sequence
<220><223> D-Peptid
<400> 14
   Arg Pro Arg Thr Arg Leu His Thr His Arg Asn Arg Asn Trp Asn Arg
   1               5                   10                  15
   Pro Arg Thr Arg Leu His Thr His Arg Asn Arg
               20                  25
<210> 15
   <211> 24
   <212> PRT
   <213> artificial sequence
<220><223> D-Peptid
<220>
   <223> doppel-D3-freie-Ntermini (rprtrlhthrnr)2-PEG3
<400> 15
   Arg Pro Arg Thr Arg Leu His Thr His Arg Asn Arg Arg Pro Arg Thr
   1 5 10 15
   Arg Leu His Thr His Arg Asn Arg
   20
<210> 16
   <211> 24
   <212> PRT
   <213> artificial sequence
<220><223> D-Peptid
<220>
   <223> doppel-D3-freie-Ctermini: PEG5-(rprtrlhthrnr)2
<400> 16
   Arg Pro Arg Thr Arg Leu His Thr His Arg Asn Arg Arg Pro Arg Thr
   1 5 10 15
   Arg Leu His Thr His Arg Asn Arg
   20
<210> 17
   <211> 11
   <212> PRT
   <213> artificial sequence
<220><223> D-Peptid
<400> 17
   Asp Ala Glu Phe Arg His Asp Ser Gly Tyr Glu
   1 5 10
<210> 18
   <211> 12
   <212> PRT
   <213> artificial sequence
<220><223> D-Peptid
<400> 18
   His His Gly His Ser Pro Asn Val Ser Gln Val Arg
   1 5 10
<210> 19
   <211> 12
   <212> PRT
   <213> artificial sequence
<220><223> D-Peptid
<400> 19
   Gly Ser Phe Ser Thr Gln Val Gly Ser Leu His Arg
   1               5                   10
<210> 20
   <211> 11
   <212> PRT
   <213> artificial sequence
<220><223> D-Peptid
<400> 20
   His Thr Gly Thr Gln Ser Tyr Val Pro Arg Leu
   1               5                   10
<210> 21
   <211> 12
   <212> PRT
   <213> artificial sequence
<220><223> D-Peptid
<400> 21
   Thr Leu Ala Tyr Ala Arg Ala Tyr Met Val Ala Pro
   1               5                   10
<210> 22
   <211> 12
   <212> PRT
   <213> artificial sequence
<220><223> D-Peptid
<400> 22
   Thr Leu Ala Tyr Ala Arg Ala Tyr Met Val Ala Pro
   1               5                   10
<210> 23
   <211> 12
   <212> PRT
   <213> artificial sequence
<220><223> D-Peptid
<400> 23
   Ala Thr Pro Gln Asn Asp Leu Lys Thr Phe Pro His
   1               5                   10
<210> 24
   <211> 12
   <212> PRT
   <213> artificial sequence
<220><223> D-Peptid
<400> 24
   Thr Gln Pro Glu Thr Asp Leu Leu Arg Val Gln Phe
   1 5 10
<210> 25
   <211> 11
   <212> PRT
   <213> artificial sequence
<220><223> D-Peptid
<400> 25
<210> 26
   <211> 12
   <212> PRT
   <213> artificial sequence
<220><223> D-Peptid
<400> 26
   Thr Phe Leu Glu Thr Gly Pro Ile Tyr Ala Asp Gly
   1 5 10
<210> 27
   <211> 12
   <212> PRT
   <213> artificial sequence
<220><223> D-Peptid
<400> 27
   Leu Val Pro Pro Thr His Arg His Trp Pro Val Thr
   1 5 10
<210> 28
   <211> 12
   <212> PRT
   <213> artificial sequence
<220><223> D-Peptid
<400> 28
   Ala Pro Pro Gly Asn Trp Arg Asn Tyr Leu Met Pro
   1 5 10
<210> 29
   <211> 12
   <212> PRT
   <213> artificial sequence
<220><223> D-Peptid
<400> 29
   Asp Asn Tyr Ser Asn Tyr Val Pro Gly Thr Lys Pro
   1               5                   10
<210> 30
   <211> 12
   <212> PRT
   <213> artificial sequence
<220><223> D-Peptid
<400> 30
   Ser Val Ser Val Gly Met Lys Pro Ser Pro Arg Pro
   1               5                   10
<210> 31
   <211> 12
   <212> PRT
   <213> artificial sequence
<220><223> D-Peptid
<400> 31
   Ser Leu Pro Asn Pro Phe Ser Val Ser Ser Phe Gly
   1               5                   10
<210> 32
   <211> 12
   <212> PRT
   <213> artificial sequence
<220><223> D-Peptid
<400> 32
   Tyr Val His Asn Pro Tyr His Leu Pro Asn Pro Pro
   1               5                   10
<210> 33
   <211> 12
   <212> PRT
   <213> artificial sequence
<220><223> D-Peptid
<400> 33
<210> 34
   <211> 12
   <212> PRT
   <213> artificial sequence
<220><223> D-Peptid
<400> 34
   Gly Ala Thr Met Lys Lys Met Asp Asp His Thr Val
   1 5 10
<210> 35
   <211> 12
   <212> PRT
   <213> artificial sequence
<220><223> D-Peptid
<400> 35
   Leu Gly Lys Thr Gln Lys Leu Ser Asp Ala His Ser
   1 5 10
<210> 36
   <211> 12
   <212> PRT
   <213> artificial sequence
<220><223> D-Peptid
<400> 36
   Asp Asp Gln Ala Arg Pro Tyr Met Ala Tyr Gly Pro
   1 5 10
<210> 37
   <211> 12
   <212> PRT
   <213> artificial sequence
<220><223> D-Peptid
<400> 37
   Gly Asp Thr Trp Val Asn Met Val Ser Met Val His
   1 5 10
<210> 38
   <211> 12
   <212> PRT
   <213> artificial sequence
<220><223> D-Peptid
<400> 38
   Gly Tyr Thr Trp Val Asn Met Val Ser Met Val His
   1 5 10
<210> 39
   <211> 12
   <212> PRT
   <213> artificial sequence
<220><223> D-Peptid
<400> 39
   Trp Thr Asn Thr Val Ala Arg Leu Ala Thr Pro Tyr
   1               5                   10
<210> 40
   <211> 12
   <212> PRT
   <213> artificial sequence
<220><223> D-Peptid
<400> 40
   Gln Thr Gln Ala Leu Tyr His Ser Arg Gln Val His
   1               5                   10
<210> 41
   <211> 12
   <212> PRT
   <213> artificial sequence
<220><223> D-Peptid
<400> 41
   Asn Ser Gln Thr Gln Thr Leu His Leu Phe Pro His
   1               5                   10
<210> 42
   <211> 12
   <212> PRT
   <213> artificial sequence
<220><223> D-Peptid
<400> 42
   His Asn Thr Ser Ala Asn Ile Leu His Ser Ser His
   1               5                   10
<210> 43
   <211> 12
   <212> PRT
   <213> artificial sequence
<220><223> D-Peptid
<400> 43
   Ser His Ile Asn Pro Thr Ser Phe Trp Pro Ala Pro
   1               5                   10
<210> 44
   <211> 12
   <212> PRT
   <213> artificial sequence
<220><223> D-Peptid
<400> 44
   Thr Phe Ser Asn Pro Leu Tyr Met Trp Pro Arg Pro
   1               5                   10
<210> 45
   <211> 12
   <212> PRT
   <213> artificial sequence
<220><223> D-Peptid
<400> 45
   Gly Pro Ser Pro Phe Asn Pro Gln Pro Thr Pro Val
   1               5                   10
<210> 46
   <211> 12
   <212> PRT
   <213> artificial sequence
<220><223> D-Peptid
<400> 46
   Phe Ser Asp His Lys Ser Pro Thr Pro Pro Pro Arg
   1               5                   10
<210> 47
   <211> 12
   <212> PRT
   <213> artificial sequence
<220><223> D-Peptid
<400> 47
   Ser Thr Ser Val Tyr Pro Pro Pro Pro Ser Ala Trp
   1               5                   10
<210> 48
   <211> 12
   <212> PRT
   <213> artificial sequence
<220><223> D-Peptid
<400> 48
   Tyr Gly Leu Pro Thr Gln Ala Asn Ser Met Gln Leu
   1 5 10
<210> 49
   <211> 12
   <212> PRT
   <213> artificial sequence
<220><223> D-Peptid
<400> 49
   His Asn Arg Thr Asp Asn Thr Tyr Ile Arg Pro Thr
   1 5 10
<210> 50
   <211> 12
   <212> PRT
   <213> artificial sequence
<220><223> D-Peptid
<400> 50
   Leu Gln Gln Pro Leu Gly Asn Asn Arg Pro Asn Ser
   1 5 10
<210> 51
   <211> 12
   <212> PRT
   <213> artificial sequence
<220><223> D-Peptid
<400> 51
   Lys Pro Glu Asp Ser Ala Ala Tyr Pro Gln Asn Arg
   1 5 10
<210> 52
   <211> 13
   <212> PRT
   <213> artificial sequence
<220><223> D-Peptid
<400> 52
   Arg Pro Glu Asp Ser Val Ile Thr Lys Thr Gln Asn Thr
   1 5 10
<210> 53
   <211> 12
   <212> PRT
   <213> artificial sequence
<220><223> D-Peptid
<400> 53
   Arg Ala Ala Asp Ser Gly cys Thr Pro Thr Lys His
   1 5 10
<210> 54
   <211> 12
   <212> PRT
   <213> artificial sequence
<220><223> D-Peptid
<400> 54
   Arg Pro Arg Thr Arg Leu His Thr His Arg Asn Thr
   1 5 10
<210> 55
   <211> 12
   <212> PRT
   <213> artificial sequence
<220><223> D-Peptid
<400> 55
   Arg Pro Arg Thr Arg Leu His Thr His Thr Asn Val
   1 5 10
<210> 56
   <211> 12
   <212> PRT
   <213> artificial sequence
<220><223> D-Peptid
<400> 56
   Arg Pro Arg Thr Arg Leu His Thr His Thr Asn Arg
   1 5 10
<210> 57
   <211> 12
   <212> PRT
   <213> artificial sequence
<220><223> D-Peptid
<400> 57
   Arg Pro Arg Thr Arg Leu His Thr His Arg Lys Gln
   1 5 10
<210> 58
   <211> 12
   <212> PRT
   <213> artificial sequence
<220><223> D-Peptid
<400> 58
   Arg Pro Arg Thr Arg Leu His Thr Leu Arg Asn Arg
   1               5                   10
<210> 59
   <211> 12
   <212> PRT
   <213> artificial sequence
<220><223> D-Peptid
<400> 59
   Arg Arg Arg Ser Pro Leu His Thr His Arg Asn Arg
   1               5                   10
<210> 60
   <211> 12
   <212> PRT
   <213> artificial sequence
<220><223> D-Peptid
<400> 60
   Leu Arg Ser Pro Arg Gln Arg Arg Ile Pro Arg Ile
   1               5                   10
<210> 61
   <211> 12
   <212> PRT
   <213> artificial sequence
<220><223> D-Peptid
<400> 61
   Arg Lys Arg Gln Leu Arg Met Thr Thr Pro Arg Pro
   1               5                   10
<210> 62
   <211> 11
   <212> PRT
   <213> artificial sequence
<220><223> D-Peptid
<400> 62
   Ser His Tyr Arg His Ile Ser Pro Ala Gln Lys
   1               5                   10
<210> 63
   <211> 24
   <212> PRT
   <213> artificial sequence
<220>
   <223> D-Peptid
<220>
   <223> doppel-D3-freie-Ntermini (rprtrlhthrnr)2
<400> 63
   Arg Pro Arg Thr Arg Leu His Thr His Arg Asn Arg Arg Pro Arg Thr
   1               5                   10                  15
   Arg Leu His Thr His Arg Asn Arg
               20
<210> 64
   <211> 24
   <212> PRT
   <213> artificial sequence
<220>
   <223> D-Peptid
<220>
   <223> doppel-D3-freie-Ctermini: (rprtrlhthrnr)2
<400> 64
   Arg Pro Arg Thr Arg Leu His Thr His Arg Asn Arg Arg Pro Arg Thr
   1               5                   10                  15
   Arg Leu His Thr His Arg Asn Arg
               20
<210> 65
   <211> 12
   <212> PRT
   <213> Artificial Sequence
<220>
   <223> D-Peptid
<400> 65
   Arg Pro Ile Thr Arg Leu Arg Thr His Gln Asn Arg
   1               5                   10
<210> 66
   <211> 12
   <212> PRT
   <213> artificial sequence
<220>
   <223> D-Peptid
<400> 66
   Arg Pro Arg Thr Arg Leu His Thr His Arg Asn Arg
   1 5 10
<210> 67
   <211> 12
   <212> PRT
   <213> Artificial Sequence
<220>
   <223> D-Peptid
<400> 67
   Pro Asn His His Arg Arg Arg Arg Arg Thr Thr Leu
   1 5 10
<210> 68
   <211> 12
   <212> PRT
   <213> Artificial Sequence
<220>
   <223> D-Peptid
<400> 68
   Arg Arg Pro Thr Leu Arg His Thr His Asn Arg Arg
   1 5 10
<210> 69
   <211> 9
   <212> PRT
   <213> Artificial Sequence
<220>
   <223> D-Peptid
<400> 69
   Arg Pro Arg Thr Arg Leu Arg Asn Arg
   1 5
<210> 70
   <211> 6
   <212> PRT
   <213> Artificial Sequence
<220>
   <223> D-Peptid
<400> 70
   Arg Pro Arg Thr Arg Leu
   1 5
<210> 71
   <211> 12
   <212> PRT
   <213> Artificial Sequence
<220>
   <223> D-Peptid
<400> 71
   Arg Pro Ile Thr Arg Leu His Thr Asp Arg Asn Arg
   1               5                   10
<210> 72
   <211> 12
   <212> PRT
   <213> Artificial Sequence
<220>
   <223> D-Peptid
<400> 72
   Arg Pro Ile Thr Thr Leu Gln Thr His Gln Asn Arg
   1               5                   10
<210> 73
   <211> 12
   <212> PRT
   <213> Artificial Sequence
<220>
   <223> D-Peptid
<400> 73
   Arg Pro Arg Thr Arg Leu Arg Thr His Gln Asn Arg
   1               5                   10
<210> 740
   <211> 12
   <212> PRT
   <213> Artificial Sequence
<220>
   <223> D-Peptid
<400> 74
<210> 75
   <211> 18
   <212> PRT
   <213> Artificial Sequence
<220>
   <223> D-Peptid
<400> 75
   Arg Pro Arg Thr Arg Leu Arg Asn Arg Arg Pro Arg Thr Arg Leu Arg Asn Arg
   1               5                   10                  15
<210> 76
   <211> 24
   <212> PRT
   <213> Artificial Sequence
<220>
   <223> D-Peptid
<400> 76
<210> 77
   <211> 12
   <212> PRT
   <213> Artificial Sequence
<220>
   <223> D-Peptid
<400> 77
   Ser Gly Trp His Tyr Asn Trp Gln Tyr Trp Trp Lys
   1               5                   10
<210> 78
   <211> 20
   <212> PRT
   <213> Artificial Sequence
<220>
   <223> D-Peptid
<400> 78
   Arg Pro Arg Thr Arg Ser Gly Trp His Tyr Asn Trp Gln Tyr Trp Trp
   1               5                   10                  15
   Lys Arg Asn Arg
               20
<210> 79
   <211> 20
   <212> PRT
   <213> Artificial Sequence
<220>
   <223> D-Peptid
<400> 79
   Pro Thr Leu Ser Gly Trp His Tyr Asn Trp Gln Tyr Trp Trp Lys Arg
   1               5                   10                  15
   Arg Arg Arg Arg
               20